**Europäisches Patentamt**

**European Patent Office**

**Office européen des brevets**

(19)

(11) Veröffentlichungsnummer: **0 199 079
B1**

# EUROPÄISCHE PATENTSCHRIFT

(12)

(45) Veröffentlichungstag der Patentschrift:
05.07.89

(21) Anmeldenummer: **86103607.7**

(22) Anmeldetag: **18.03.86**

(51) Int. Cl.⁴: **A 61 B 6/14,** A 61 B 6/00

(54) Vorrichtung zur Höhenverstellung eines an einem vertikalen Ständer gehaltertem Trägers für ein medizinisches Gerät.

(30) Priorität: **01.04.85 DE 3511907**

(43) Veröffentlichungstag der Anmeldung:
**29.10.86 Patentblatt 86/44**

(45) Bekanntmachung des Hinweises auf die Patenterteilung:
**05.07.89 Patentblatt 89/27**

(84) Benannte Vertragsstaaten:
**DE FR GB IT**

(56) Entgegenhaltungen:
**FR-A- 2 282 843
GB-A- 746 599
GB-A- 2 014 667
SE-A- 201 217
US-A- 3 575 368**

(73) Patentinhaber: **Siemens Aktiengesellschaft Berlin und München, Wittelsbacherplatz 2, D-8000 München 2 (DE)**

(72) Erfinder: **Günther, Werner, Odenwaldstrasse 20, D-6140 Bensheim (DE)**
Erfinder: **Schmitt, Heinrich, Drussusstrasse 9, D-6140 Bensheim (DE)**

**Beschreibung**

Insbesondere bei Röntgendiagnostikgeräten, die im zahnmedizinischen Bereich zur Erstellung von Panorama-Röntgenaufnahmen vom Kiefer eines Patienten eingesetzt werden, besteht das Problem, die aus Röntgengenerator und Röntgenfilmhalter bestehende Dreheinheit in einem bestimmten Bereich stufenlos in der Höhe verstellen zu können, um so die Dreheinheit unterschiedlichen Patientengrößen (Kind/Erwachsener) oder unterschiedlichen Aufnahmetechniken (am sitzenden oder stehenden Patienten) anpassen zu können.

Bei einem bekannten solchen Gerät (Prospekt M-D 80/1361 ORTHOPANTOMOGRAPH 10; WS 08 832) ist die Dreheinheit auf einem Laufschlitten montiert, der an einem ortsfesten Ständer verstellbar gehaltert ist. Aus Stabilitätsgründen besteht der Ständer aus einteiligen, im Querschnitt rechteckigen Rahmenteilen, an deren schmalen Seiten über Schienen der Laufschlitten geführt ist. Die Rahmenteile bilden einen Hohlraum, in dem über einen Seilzug Gewichte aus Stahl oder Blei als Gewichtsausgleich für die Dreheinheit angeordnet sind. Wegen des relativ hohen, vom Träger aufzunehmenden Gerätegewichts ist eine äußerst präzise, insbesondere spielfreie, Führung vorzusehen, was natürlich mit einem entsprechenden Kostenaufwand verbunden ist. Der Gewichtsausgleich ist zwar sehr einfach, nimmt aber relativ viel Platz in Anspruch. Darüber hinaus ergeben sich wegen des mit etwa 90 kg anzusetzenden Gegengewichts Probleme beim Aufstellen und Montieren.

Ein demgegenüber vereinfachter Ständeraufbau ist aus GB-A-746 599 bekannt. Der Ständer besteht dort aus einem Rahmen mit zwei beabstandeten senkrechten [-Profilen, zwischen denen ein Laufwagen höhenverstellbar geführt ist. Hierzu sind in den [-Profilen Rundstangen befestigt, an denen sich am Laufwagen angeordnete Laufrollen abwälzen können. Die Kombinationen Rundstange/Laufrollen ist trotz des vereinfachten Ständeraufbaus noch vergleichsweise kostenaufwendig. Nachdem die Laufrollen in den Führungskanal des Ständerprofils hineinragen, muß der Führungskanal relativ breit, das Profil also praktisch nach einer Seite hin offen ausgebildet sein. Bei der bekannten Ständerkonstruktion sind keine Maßnahmen zur Gewichtskompensation des Laufschlittens angegeben. Solche sind beispielsweise in der US-A-3 575 368 aufgezeigt. Bei dem dort beschriebenen Röntgengerät ist eine Gewichtsausgleichsvorrichtung, ähnlich wie bei dem eingangs erwähnten Gerät, in einer relativ hohen Standsäule untergebracht, und zwar in Form einer Druckfeder und eines über eine Kurvenseilscheibe mit veränderbarem Durchmesser geführten Zugseils. Für den Laufwagen ist eine Fallsicherung in Form einer trapezförmigen Platte, an der das Zugseil aufgehängt ist, vorgesehen, deren Kanten sich beim Reißen des Seils an Führungswänden eines Stativs festhalten

Ziel der vorliegenden Erfindung ist es, demgegenüber eine verbesserte und vereinfachte Höhenverstellvorrichtung mit Gewichtsausgleich anzugeben, mit der sich insbesondere die erläuterten Nachteile vermeiden und die Herstellungskosten verringern lassen.

Zur Lösung der gestellten Aufgabe werden die im Anspruch 1 angegebenen Maßnahmen vorgeschlagen. Vorteilhafte Ausgestaltungen und Weiterbildungen der Erfindung sind in den Unteransprüchen enthalten.

Die vorgeschlagene Vorrichtung läßt sich relativ einfach, trotzdem aber ausreichend stabil erstellen und nimmt wenig Platz in Anspruch, da die Teile zum Gewichtsausgleich von dem ohnehin vorhandenen verstellbaren Träger aufgenommen werden bzw. teilweise im Tragrohr angeordnet sind. Ein weiterer Vorteil ist darin zu sehen, daß sich mit relativ einfachen Mitteln eine verdeckte, gegen Schmutz geschützte Führung erzielen läßt. Ein Verstellen des Laufschlittens kann von Hand oder auch motorisch erfolgen. Letzteres ist besonders vorteilhaft in einer Ausführungsform, bei der eine der Umlenkrollen mit einem motorischen Antrieb und einer Bremse, vorzugsweise einer Magnetbremse, gekuppelt ist.

Anhand der Zeichnung wird ein Ausführungsbeispiel der Erfindung näher erläutert:

Es zeigen:

Fig. 1 die erfindungsgemäße Einrichtung in einer schaubildlichen Darstellung,

Fig. 2 und 3 Alternativlösungen für einen Teil der Einrichtung nach Figur 1,

Fig. 4 die Rohrrahmenkonstruktion nach Figur 1 alleine,

Fig. 5 eine Prinzipskizze für die Verstelleinrichtung des Laufschlittens,

Fig. 6 Einzelheiten betreffend Aufbau und Montage der Rohrkonstruktion und einer Fallsicherung für den Laufschlitten,

Fig. 7 eine Alternativlösung zur Führung des Laufschlittens,

Fig. 8 einen Schnitt entlang der Linie VIII/VIII in Figur 5,

Fig. 9 bis 11 eine weitere Ausführungsform einer gewichtskompensierten Verstelleinrichtung für den Laufschlitten.

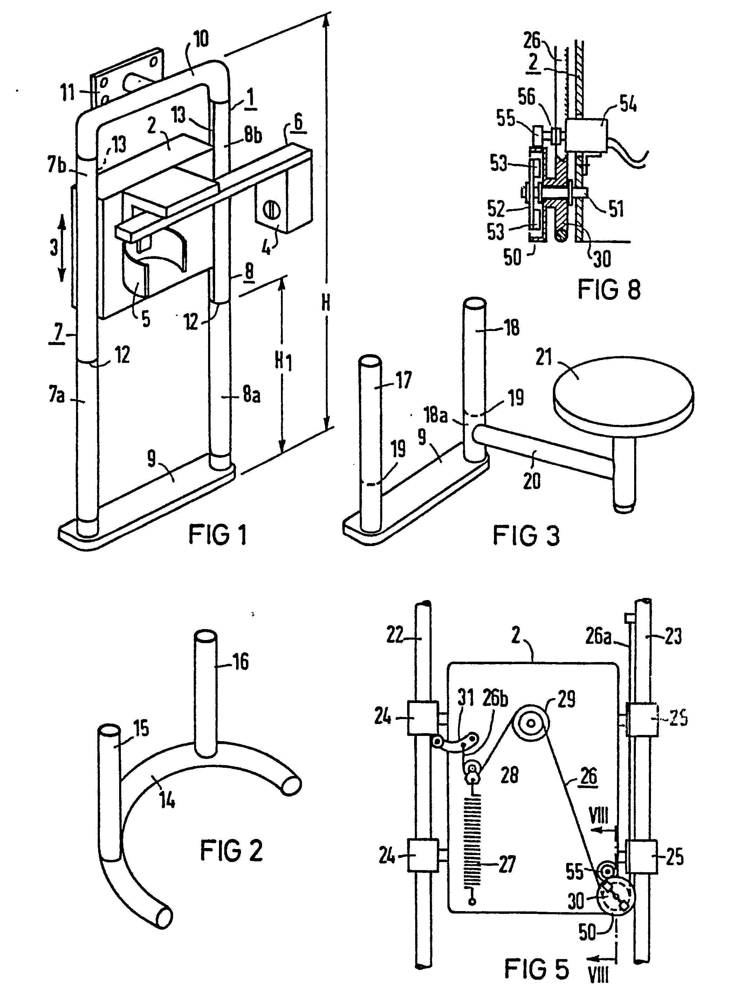

Die Figur 1 zeigt in einer schaubildlichen Darstellung eine zahnärztliche Röntgendiagnostikeinrichtung zur Erstellung von Panorama-Röntgenaufnahmen. Die Einrichtung besteht aus einem Ständer oder Stativ 1, an dem ein Laufschlitten 2 in Richtung des Pfeils 3 verstellbar gehaltert ist. Am Laufschlitten 2 ist eine aus Röntgengenerator 4 und Röntgenfilmhalter 5 bestehende Dreheinheit 6 in üblicher Weise gehaltert. Nachdem die Halterung der Dreheinheit für die Erfindung unbedeutend ist, wird diese nicht näher erläutert.

Der Ständer 1 besteht aus einem Rohrrahmen mit zwei parallelen vertikalen Tragrohren 7, 8, die an ihren Enden durch Querstreben 9 und 10 miteinander verbunden sind. Die Querstreben 9, 10 sind U-förmig ausgebildet; die deckenseitige Querstrebe 10 ist mit einem an einer Stellwand befestigbaren Abstandsglied 11 versehen.

Die Tragrohre 7, 8 sind an der mit 12 bezeichneten Stelle quergeteilt und lassen sich so in zwei Rohrabschnitte unterteilen, in einen unteren Rohrabschnitt mit den Rohren 7a, 8a und in einen oberen Rohrabschnitt mit den Rohren 7b, 8b. Die Rohre des unteren Rohrabschnittes weisen einen geschlossenen (kreisförmigen) Querschnitt auf, hingegen die Rohre 7b, 8b des oberen Rohrabschnittes mit einem Längsschlitz 13 versehen sind, aus dem Teile einer später noch näher erläuterten Führungseinrichtung zur Führung des Laufschlittens 2 vorstehen. Die Querteilung 12 erfolgt an einer Stelle, die etwa der untersten verlangten Höheneinstellung der Dreheinheit 6 entspricht; bei der heute üblichen Einstelltechnik beträgt diese Höhe ($H_1$), gerechnet vom Fußboden an, etwa 1 m bis 1,5 m bezogen auf eine Gesamtraumhöhe (H) von etwa 2,40 m.

Durch die Querteilung der Tragrohre ist es leicht möglich, anstelle der gezeigten, an einer Stellwand montierbaren Ständereinheit eine freistehende Ständereinheit vorzusehen oder im unteren Rohrabschnitt Zusatzgeräte, wie z.B. einen Patientenstuhl, zu integrieren. In den Figuren 2 und 3 sind zwei solcher Möglichkeiten aufgezeigt. Bei der einen Variante wird das in Figur 2 gezeigte Fußteil mit dem C-förmig gebogenen Standfuß 14 und den beiden Rohren 15 und 16 anstelle des unteren Rohrabschnittes mit den Rohren 7a, 8a und der Querstrebe 9 (Figur 1) gesetzt. Bei der Alternativlösung gemäß Figur 3 sind die beiden Standrohre 17, 18 an der mit 19 bezeichneten Stelle nochmals quergeteilt; das untere Rohrteil 18a des Standrohres 18 ist mit einem Ausleger 20 versehen, an dem ein Patientensitz 21 befestigt ist. Das Rohrteil 18a ist an der Querstrebe 9 drehbar gelagert, so daß der Sitz 21 um die Lagerachse des Rohrteils 18a geschwenkt werden kann.

Aufgrund des rohrförmigen Aufbaus des Ständers, welcher nicht auf die Verwendung zylindrischer Rohre, wie dargestellt, beschränkt ist, ist es möglich, unter Verwendung weitgehend gleicher Bauteile dem Kunden eine Vielfalt an verschiedenen Geräteausführungen anzubieten. Durch die sinnvolle Querteilung der vertikalen Tragrohre ergeben sich gegenüber den bisher bekannten Konstruktionen erheblich kleinere Verpackungsgrößen. Weitere Vorteile ergeben sich aus der nachfolgenden näheren Beschreibung der Konstruktion.

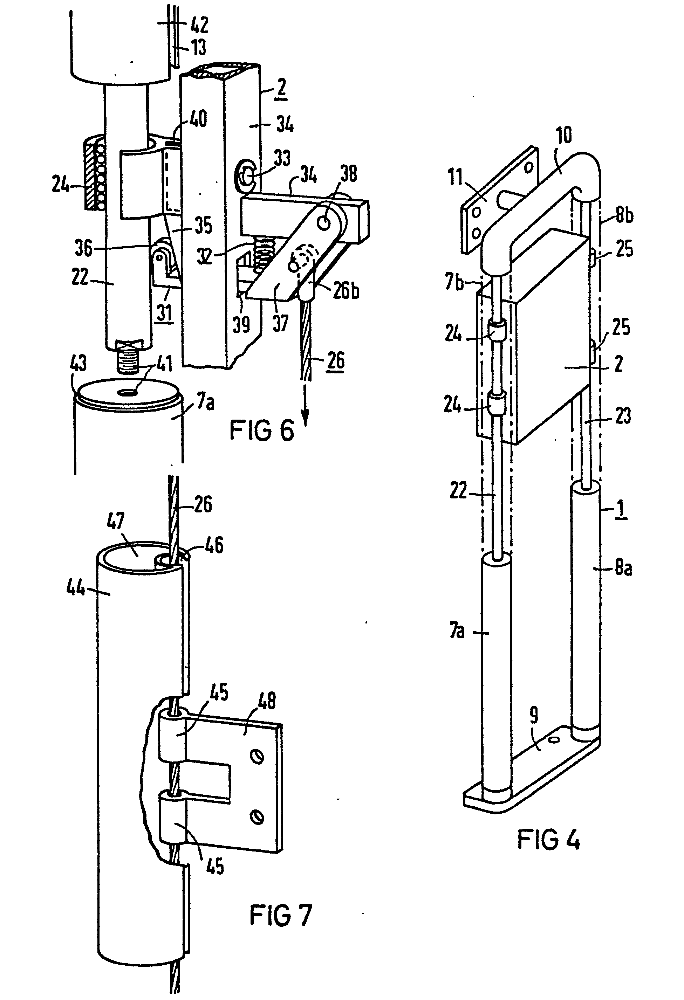

Die Figur 4 zeigt Einzelheiten des Ständeraufbaus. Die Rohre 7b, 8b des oberen Rohrabschnittes umschließen Führungsstangen 22, 23, auf denen Buchsen 24, 25 entlanggleiten. Die Buchsen 24, 25 sind mit dem Laufschlitten 2 verbunden, wobei die Buchsen 25 fest und die Buchsen 24 mit Spiel mit dem Laufschlitten 2 verbunden sind. Näheres ist aus den nachfolgenden Figuren ersichtlich. Die Führungsstangen 22, 23 sind einerseits mit den Schenkeln der oberen Querstrebe 10 und andererseits mit den Rohren 7a, 8a verbunden; zweckmäßigerweise kann diese Verbindung aus einer Schraubverbindung bestehen, wie diese beispielsweise in Figur 6 gezeigt ist.

Um das Gewicht der am Laufschlitten 2 befestigten Dreheinheit 6 kompensieren zu können, ist im Laufschlitten eine an sich bekannte Gewichtsausgleichsvorrichtung vorgesehen, welche in Figur 5 vom Prinzip her aufgezeigt ist. Die Ausgleichsvorrichtung enthält ein Zugseil 26, welches über eine beweglich geführte und mittels Zugfeder 27 belastete Umlenkrolle 28, eine Seilscheibe 29 und eine am Laufschlitten 2 ortsfest gelagerte weitere Umlenkrolle 30 geführt ist. Das eine Ende 26a des Zugseils 26 ist an der Führungsstange 23, das andere Ende 26b an einer Klemmeinrichtung 31 befestigt. Die sich bei Verstellung des Schlittens ändernde Federkraft wird durch die Seilscheibe 29 kompensiert, so daß das Gewicht des Laufschlittens und das der an ihm gehaltenen Dreheinheit 6 in jeder Höhenlage ausgeglichen ist.

Die Klemmeinrichtung 31 ist Teil einer Fallsicherung, die anhand der Figur 6 näher erläutert wird.

Wie bereits dargelegt, ist der Laufschlitten 2 mittels der Führungsbuchsen 24, 25 in den Führungsstangen 22, 23 längsverschiebbar geführt. In Figur 6 ist hierzu der linke Teil der Führung näher dargestellt. Als Führungsbuchse 24 ist eine Kugelbuchse vorgesehen, an der ein Bolzen 33 befestigt ist, der mit dem Rahmen 34 des Laufschlittens 2 fest verbunden ist. Die Verbindung erfolgt auf der anderen Seite mit Spiel, d.h. der Bolzen und damit die Kugelbuchse 25 kann sich dort in Axialrichtung des Bolzens geringfügig zum Rahmen 34 hin bewegen, wodurch ein Verkanten des Laufschlittens in der Führung vermieden wird.

Die Führungsbuchse 24 enthält einen mit einer schrägen Fläche versehenen Keil 35, mit dem eine Klemmrolle 36 zusammenwirkt. Die Klemmrolle 36 ist an einem Kipphebel 37 gelagert, der wiederum mittels Lager 38 am Rahmen 34 kippbar gelagert ist. Das eine Ende 26b des Zugseils 26 ist am Kipphebel 37 eingehängt. Bei dem normalerweise vorhandenen Zug am Seil 26 wird der Kipphebel 37 gegen einen Anschlag 39 am Rahmen 34 gedrückt; in dieser Stellung hat die Rolle 36 keinen Kontakt mit der Führungsstange 22. Für den Fall, daß der Seilzug nicht gewährleistet ist, wird bei einer dadurch verbundenen Abwärtsbewegung des gesamten Laufschlittens die Klemmrolle 36 zwischen der Schräge des Keiles 35 und der Führungsstange 22 eingeklemmt, wodurch der Laufschlitten sofort arretiert wird. Eine zwischen Rahmen 34 und Kipphebel 37 angeordnete Zugfeder 32 unterstützt den Einklemmvorgang bei einem eventuell auftretenden Seilriß.

In Betrachtung der Figur 5 ist ersichtlich, daß das Seil 26 von der Umlenkrolle 30 aus über die beiden Führungsbuchsen 25 geführt ist. Hierzu sind entsprechende Freiräume in Form von z.B. Ausnehmungen, Durchbrüchen oder Schlitzen, wie in Figur 6 mit der Position 40 bezeichnet, vorhanden.

In Figur 6 ist weiterhin die Verbindung der Führungsstangen 22 (bzw. 23) mit den Tragrohren 7a (bzw. 8a) der unteren Rohrabschnitte dargestellt. Anstelle der gezeigten, aus Gewindezapfen und Gewindebohrung bestehenden Schraubverbindung (Position 41) kann auch eine andere adäquate Verbindung vorgesehen werden.

Im Bereich der Rohrabschnitte 7b, 8b wird die Führungseinrichtung von einer äußeren, den Längsschlitz 13 enthaltenden Hülle 42 umschlossen. Diese, in Figur 6 oben links dargestellte Hülle wird zentriert durch einen kleinen Ansatz 43 (links unten in Figur 6) im korrespondierenden Tragrohr.

Die Hülle 42 kann aus einem gerollten Blech gefertigt sein; sie kann aber gemäß einer vorteilhaften Ausgestaltung der Erfindung so ausgebildet sein, daß sie gleichsam die Lagerung für die Führung des Laufschlittens bildet. Eine solche Alternativlösung ist in Figur 7 dargestellt. Hülle und Standrohr bestehen hier aus einem entsprechend dem dargestellten Profil gerollten Teil 44. Das Profil ist so gestaltet, daß ein den Führungsteilen 45 entsprechender zylindrischer Führungskanal 46 gebildet wird. Der verbleibende Hohlraum 47 kann ausgeschäumt werden, wodurch der Führungskanal 46 eine zusätzliche Steifigkeit bekommt. Der Laufschlitten ist bei dieser Alternativlösung an einem mit den Führungsteilen 45 verbundenen Trägerteil 48 befestigt. Zur Führung des Zugseils sind die Führungsteile 45 vorteilhafterweise als Buchsen ausgebildet.

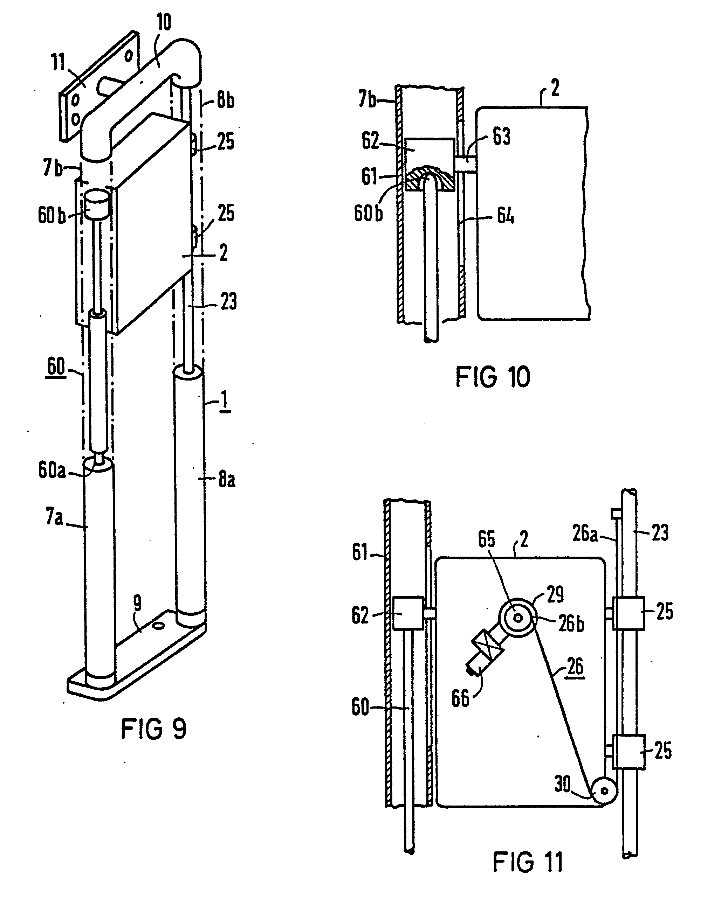

Wie aus Figur 8, die einen Schnitt entlang der Linie VIII/VIII in Figur 5 zeigt, ersichtlich, ist die Umlenkrolle 30 mit einer topfartig ausgebildeten Trommel 50 aus weichmagnetischem Material verbunden. Trommel 50 und Umlenkrolle 30 sind auf einer am Laufschlitten 2 befestigten Achse 51 drehbar gehalten. Auf der Achse 51 ist ein Hebel 52 befestigt, an dem zwei Elektromagnetspulen 53 angeordnet sind, welche mit der Trommel 50 einer Elektromagnetbremse zum Festhalten des Laufschlittens 2 — und damit der von ihm getragenen Dreheinheit 6 — in einer beliebig einstellbaren Höhenlage dient. Das Ein- und Abschalten der beiden Topfmagnete 53 kann in bekannter Weise durch einen in einem nicht dargestellten Stromkreis angeordneten Schalter oder Taster erfolgen.

Die Trommel 50 dient gleichsam auch zum motorischen Verstellen des Laufschlittens 2. Hierzu liegt im äußeren Trommelrand ein von einem Elektromotor 54 angetriebenes Reibrad 55 an. Eine zwischen Antriebsmotor 54 und Reibrad angeordnete Rutschkupplung 56 erlaubt auch eine Verstellung des Laufschlittens von Hand.

Die Figuren 9 bis 11 zeigen eine weitere vorteilhafte Ausführung einer Verstelleinrichtung mit Gewichtskompensation für den Laufschlitten 2. Anstelle der Zugfeder (Position 27 in Figur 5) ist als Kraftglied eine Gasfeder 60 vorgesehen. Die Gasfeder 60 ist in einem Hüllrohr 61 des linken oberen Rohrabschnittes 7b so angeordnet, daß sich das eine Ende 60a, im Ausführungsbeispiel das freie Ende des Zylinders der Gasfeder, am stirnseitig geschlossenen Ende des unteren Rohrabschnittes 7a abstützt und das andere Ende 60b der Gasfeder, im Ausführungsbeispiel das Ende der Kolbenstange, sich an einer Gleitbuchse 62 abstützt. Die Gleitbuchse 62 ist im Hüllrohr 61 in geeigneter Weise geführt und mittels eines Mitnahmebolzens 63 mit dem Laufschlitten (ähnlich wie in Figur 6 gezeigt) verbunden. Das Hüllrohr 61 enthält entweder einen durchgehenden oder nur über den Verstellbereich sich erstreckenden Schlitz 64 mit einer praktisch nur der Bolzenstärke entsprechenden Schlitzbreite. Die Führung des Laufschlittens 2 auf der gegenüberliegenden Seite erfolgt, wie bereits zuvor beschrieben, mittels zweier Kugelbuchsen 25.

Damit negative Einflüsse der Gasfeder, wie Anstieg der Federkennlinie im ein- bzw. ausgefahrenen Zustand, Temperaturschwankungen od. dgl., kompensiert werden können, übernimmt die Gasfeder nur einen Teil der Gewichtskompensation. Zusätzlich sind im Laufschlitten 2 ein zweites Kraftglied in Form eines Elektromotorantriebs (Pos. 66) und eine mit dem Antrieb gekuppelte Seilführung vorgesehen. Diese Anordnung ist in Figur 11 im Prinzip dargestellt. Das Seilende 26b ist an einer Seilrolle 65 befestigt, welche von einem DC-Getriebemotor 66 angetrieben wird. Die Gasfeder 60 ist so ausgelegt, daß sie eine etwas geringere Gegenkraft erzeugt als zur Kompensation des Gewichtes des Laufwagens 2 notwendig, d.h. das Gewicht des Laufwagens ist geringfügig größer als die maximale Gegenkraft, die durch die Gasfeder 60 erzeugt wird. Der Getriebemotor 66 wiederum ist so ausgelegt, daß er lediglich die Differenzkraft zwischen der Gewichtskraft des Laufwagens und der Gegenkraft der Gasfeder aufzubringen hat.

Die vorbeschriebene Anordnung der Gasfeder hat u.a. den Vorteil, daß sich eine vergleichsweise einfache Gewichtskompensation für die Höhenverstelleinrichtung erzielen läßt, bei der auf eine Fallsicherungseinrichtung verzichtet werden kann. Wenngleich die Anordnung der Gasfeder in dem einen Tragrohr als besonders vorteilhaft anzusehen ist, weil sich dadurch keine vorstehenden Teile ergeben, so ist dies nicht zwingend erforderlich; denkbar und im Rahmen der Erfindung liegt es deshalb auch, die Gasfeder außerhalb des Tragrohres anzulenken, indem man z.B. das eine Ende der Gasfeder direkt am Laufwagen und das andere Ende am ortsfesten Tragrohr abstützt.

**Patentansprüche**

1. Vorrichtung zur Höhenverstellung eines Trägers (2) für ein medizinisches Gerät, insbesondere für eine aus Röntgengenerator (4) und Filmhalter (5) bestehende Dreheinheit (6) eines Orthopantomographiegerätes, wobei der Träger (2) an einem aus zwei vertikalen, parallel in einem Abstand zueinander angeordneten Tragelementen (7, 8) bestehenden Ständer (1) höhenverstellbar gehaltert ist, und wobei die Tragelemente (7, 8) längs verlaufende Kanäle für Führungselemente (22 bis 25, 45, 62) enthalten, an denen der Träger (2) gehalten ist, dadurch gekennzeichnet, daß als Tragelemente an sich am Umfang geschlossene, zumindest aber im Verstellbereich des Trägers (2) mit Längsschlitzen (13, 64) versehene Rohre (7, 8) vorgesehen sind, daß die Führungselemente (24, 25, 45, 62) in den Rohren (7, 8) gleitend geführt und mittels durch die Schlitze (13, 64) hindurchgreifende Mitnehmerelemente (33, 63) mit dem Träger (2) verbunden sind, und daß für den Träger eine Gewichtsausgleichsvorrichtung vorgesehen ist, welche ein Kraftglied [Zugfeder (27), Gasfeder (60), Antriebsmotor (66)] enthält welches entweder in einem der Rohre (7, 8) und/oder am Träger (2) angeordnet ist und daß die Gewichtsausgleichsvorrichtung ferner ein Zugseil (26) enthält, dessen eines Ende (26a) an einem der Rohre (7, 8) und dessen anderes Ende (26b) am Träger (2) befestigt ist und welches dazwischen über wenigstens eine am Träger (2) angeordnete Umlenkrolle (29, 30) zu dem am Träger (2) angeordneten Kraftglied (27, 66) geführt ist.

2. Vorrichtung nach Anspruch 1, dadurch gekennzeichnet, daß die Tragrohre (7, 8) zumindest eine die Rohrlänge etwa halbierende Querteilung (12) aufweisen, an der die geteilten Rohrabschnitte (7a, 7b; 8a, 8b) bei Montage miteinander verbunden werden.

3. Vorrichtung nach Anspruch 2, dadurch gekennzeichnet, daß die beiden Tragrohre (7, 8) aus einem mit einer bodenseitigen Querverstrebung (9) verbundenen und bis zur Querteilung (12) sich erstreckenden unteren Rohrabschnitt (7a, 8a) mit Rohren mit im Querschnitt geschlossenem Profil und einem sich daran anschließenden und mit dem unteren Rohrabschnitt (7a, 8a) und einer deckseitigen Querverstrebung (10) verbundenen oberen Rohrabschnitt (7b, 8b) mit Rohren (42) mit im Querschnitt einseitig offenem, den Längsschlitz (13) bildendem Profil bestehen.

4. Vorrichtung nach Anspruch 1, dadurch gekennzeichnet, daß von dem mit der bodenseitigen Querverstrebung (9) verbundenen Rohrabschnitt (7a, 8a) das eine Rohr (8a) am Standfuß (9) fest und das andere (7a) in der Höhe einstellbar befestigt ist.

5. Vorrichtung nach Anspruch 1, dadurch gekennzeichnet, daß die Rohre des mit Längsschlitzen (13) versehenen Rohrabschnittes (7b, 8b) jeweils eine, vorzugsweise zentrisch angeordnete, mit boden- und deckseitigen Elementen (7a, 8a, 10) verbindbare Führungsstange (22, 23) enthalten, auf der mit dem Träger (2) verbundene Führungsbuchsen (24, 25) entlanggleiten, und daß die Führungsstangen (22, 23) von einem den Längsschlitz (13) enthaltenden und im Außendurchmesser den sich daran anschließenden Rahmenteilen (7a, 8a; 10) entsprechenden Hüllrohr (42) umgeben ist.

6. Vorrichtung nach Anspruch 1, dadurch gekennzeichnet, daß die mit Längsschlitzen (13, 64) versehenen Rohrabschnitte (7b, 8b) durch ein Hüllrohr (44, 61) gebildet werden, welches so gestaltet ist, daß es einen Führungskanal bildet, in dem ein Führungsteil (45, 62), an dem der Träger (2) befestigt ist, entlanggleiten kann.

7. Vorrichtung nach Anspruch 6, dadurch gekennzeichnet, daß das Hüllrohr (44) aus einem gerollten Blech besteht.

8. Vorrichtung nach Anspruch 5 oder 6, dadurch gekennzeichnet, daß die Führungselemente (23, 24, 45, 62) zur Führung des Trägers (2) an der einen Seite des Trägers fest und an der anderen Seite mit dem Spiel mit dem Träger (2) verbunden sind.

9. Vorrichtung nach Anspruch 1, dadurch gekennzeichnet, daß der Träger (2) mit einer Fallsicherungsvorrichtung (35 bis 38) versehen ist, die aus einem zwischen Führungsrohr (22) und einer mit dem Laufschlitten (2) verbundenen schrägen Fläche (35) einklemmbaren, mit dem einen Ende (26b) des Zugseils (26) verbundenen Klemmkörper (36) besteht, der infolge der Federwirkung der Zug- oder Druckfeder (26) bei vorhandenem Seilzug außer und bei nicht vorhandenem Seilzug in Klemmeingriffsposition kommt.

10. Vorrichtung nach Anspruch 1, dadurch gekennzeichnet, daß als Kraftglied eine im Träger (2) angeordnete Zugfeder (27) vorgesehen ist, welche ein über Umlenkrollen (29, 50) geführtes Zugseil (26) mit einer der Gewichtskompensation entsprechenden Zugkraft spannt, und daß eine der Umlenkrollen (29, 30) mit einem Fremdantrieb (50, 54, 55, 66) gekuppelt ist.

11. Vorrichtung nach Anspruch 10, dadurch gekennzeichnet, daß die Umlenkrolle (30) mit einer Bremseinrichtung (50 bis 53) gekuppelt ist.

12. Vorrichtung nach Anspruch 11, dadurch gekennzeichnet, daß Antrieb und Bremseinrichtung über eine gemeinsame, mit der Umlenkrolle (30) verbundene Trommel (50) erfolgt.

13. Vorrichtung nach Anspruch 12, dadurch gekennzeichnet, daß an der Umlenkrolle (30) eine Trommel (50) befestigt ist, an deren äußerem Rand ein von einem Antriebsmotor (54) angetriebenes Reibrad (55) anliegt, und daß stirnseitig der Trommel (50) auf einem gegenüber dieser nicht drehbaren Träger (52) zwei Elektromagnete (53) befestigt sind, die mit korrespondierenden, aus weichmagnetischem Werkstoff bestehenden Teilen der Trommel (50) zusammenwirken.

14. Vorrichtung nach Anspruch 1, dadurch gekennzeichnet, daß als Kraftglied ein eine Seilrolle (65) antreibender Getriebemotor (66) vorgesehen ist.

15. Vorrichtung nach Anspruch 1, dadurch gekennzeichnet, daß ein weiteres Kraftglied in Form einer Gasfeder (60) vorgesehen ist, deren eines Ende am Ständer (7) und deren anderes Ende am Laufschlitten (2) angreift.

16. Vorrichtung nach Anspruch 15, dadurch gekennzeichnet, daß die Gasfeder (60) in einem Hüllrohr (61) des einen Rohrabschnittes (7b) angeordnet ist und sich mit ihrem einen Ende an einer im Hüllrohr (61) gleitend geführten und mit dem Laufschlitten (2) verbundenen Buchse (62) abstützt.

**Claims**

1. An apparatus for adjusting the height of a support (2) for a medical device, in particular for a rotary unit (6) comprising an X-ray generator (4) and a film holder (5) of an orthopantomography device, wherein the support (2) is mounted so as to be adjustable in height on a stand (1) comprising two vertical parallel supporting elements (7, 8) spaced from one another, and wherein the supporting elements (7, 8) include longitudinally extending passages for guide elements (22 to 25, 45, 62) to which the support (2) is attached, characterised in that the supporting elements comprise tubes (7, 8) closed on the periphery but provided with longitudinal slits (13, 64) at least in the adjusting region of the support (2); that the guide elements (24, 25, 45, 62) are guided to slide in the tubes (7, 8) and are connected to the support (2) by means of carrier elements (33, 63) engaging through the slits (13, 64); and that a counter-balance device is provided for the support which includes a power element [tension spring (27), pneumatic spring (60), drive motor (66)] which is arranged either in one of the tubes (7, 8) and/or on the support (2) and that the counter-balance device further comprises a traction line (26) whose one end (26a) is attached to one of the tubes (7, 8) and whose other end (26b) is attached to the support (2) and which is guided therebetween over at least one guide roller (29, 30) arranged on the support (2) to the power element (27, 66) arranged on the support (2).

2. An apparatus according to claim 1, characterised in that the supporting tubes (7, 8) have at least one transverse division (12) approximately halving the length of the tube at which the divided tubular sections (7a, 7b; 8a, 8b) are joined to one another when assembled.

3. An apparatus according to claim 2, characterised in that the two supporting tubes (7, 8) comprise a lower tube section (7a, 8a) connected to a bottom cross strut (9) and extending up to the transverse division (12) and having tubes with closed cross-sectional profiles, and an upper tube section (7b, 8b) adjacent thereto and connected to the lower tube section (7a, 8a) and to a top cross strut (10) and having tubes (42) with a cross-section open at one side to form the longitudinal slit (13).

4. An apparatus according to claim 1, characterised in that of the tube section (7a, 8a) connected to the bottom cross strut (9), one tube (8a) is securely fixed to the base (9) and the other tube (7a) is attached so that its height can be adjusted.

5. An apparatus according to claim 1, characterised in that the tubes of the tube section (7b, 8b) provided with longitudinal slits (13) each have a guide rod (22, 23), preferably arranged centrally, which can be connected to the bottom and top elements (7a, 8a; 10), along which guide sleeves (24, 25) connected to the support (2) slide, and that the guide rods (22, 23) are surrounded by a tubular jacket (42) including the longitudinal slit (13) and with its outer diameter corresponding to the frame parts (7a, 8a; 10) connected thereto.

6. An apparatus according to claim 1, characterised in that the tube sections (7b, 8b) provided with longitudinal slits (13, 64) are formed by a jacket tube (44, 61) which is shaped so that it forms a guiding passage in which a guiding part (45, 62), to which the support (2) is attached, can slide along.

7. An apparatus according to claim 6, characterised in that the jacket tube (44) consists of a rolled sheet.

8. An apparatus according to claim 5 or claim 6, characterised in that the guiding elements (23, 24, 45, 62) for guiding the support (2) are securely connected to one side of the support and with play to the other side of the support (2).

9. An apparatus according to claim 1, characterised in that the support (2) is provided with a falling safety device (35 to 38) which consists of a clamping part (36), connected to one end (26b) of the traction line (26), which can be clamped in between the guide tube (22) and an inclined surface (35) connected to the carriage (2), and which, as a result of the spring action of the tension or pressure spring (26), comes into a clamping engagement position when there is no tension on the line, and a disengaged position when there is tension on the line.

10. An apparatus according to claim 1, characterised in that as a power element a tension spring (27) arranged in the support (2) is provided which tensions the traction line (26) guided over guide rollers (29, 30) with tractive force corresponding to the weight compensation and that one of the guide rollers (29, 30) is coupled with a separate drive (50, 54, 55, 66).

11. An apparatus according to claim 10, characterised in that the guide roller (30) is coupled with a braking device (50 to 53).

12. An apparatus according to claim 11, characterised in that the drive and braking devices operate by way of a common drum (50) connected to the guide roller (30).

13. An apparatus according to claim 12, characterised in that a drum (50) is connected to the guide roller (30) and has a friction wheel (55), driven by a drive motor (54), bearing on its outer edge, and that two electromagnets (53) are attached at the front of the drum (50) on a support (52) non-rotatable relative thereto and cooperate with two corresponding parts of the drum (50) that consist of soft-magnetic material.

14. An apparatus according to claim 1, characterised in that a drive motor (66) driving a rope pulley (65) is provided as a power element.

15. An apparatus according to claim 1, characterised in that a further power element is provided in the form of a pneumatic spring (60) whose one end engages with the base (7) and whose other end engages with the carriage (2).

16. An apparatus according to claim 15, characterised in that the pneumatic spring (60) is arranged in a jacket tube (61) of the one tube section (7b) and is supported with its one end on a sleeve (62) guided in the jacket tube (61) and connected to the carriage (2).

## Revendications

1. Dispositif de réglage en hauteur d'un support (2) pour un appareil médical, notamment pour une unité rotative (6) constituée par un générateur radiologique (4) et un support de film (5), d'un appareil d'orthopantomographie, dans lequel le support (2) est maintenu de manière à être réglable en hauteur sur un pied (1) constitué par deux éléments verticaux de support (7, 8) disposés à distance parallèlement l'un à l'autre, et dans lequel les éléments de support (7, 8) comportent des conduits longitudinaux pour des organes de réglage (22 à 25, 45, 62), sur lesquels le support (2) est maintenu, caractérisé par le fait qu'il est prévu, comme éléments de support, des tubes (7, 8) fermés sur leur pourtour, mais comportant au moins, dans la zone de réglage du support (2), des fentes longitudinales (13, 34), que les organes de guidage (24, 25, 45, 62) sont guidés de manière à pouvoir glisser dans les tubes (7, 8) et sont reliés au support (2) au moyen d'éléments d'entraînement (33, 63) s'engageant dans les fentes (13, 64), et que pour le support il est prévu un dispositif formant contrepoids, qui contient un élément d'application d'une force [ressort de traction (27), ressort pneumatique (60), moteur d'entraînement (66)], qui est disposé dans l'un des tubes (7, 8) et/ou sur le support (2), et que le dispositif formant contrepoids contient en outre un câble de traction (26), dont une extrémité (26b) est fixée à l'un des tubes (7, 8) et dont l'autre extrémité (26b) est fixée au support (2) et qui circule entre ses extrémités, sur au moins un galet de renvoi (29, 30) disposé sur le support (2) tout en étant raccordé à l'élément d'application d'une force (27, 66), disposé sur le support (2).

2. Dispositif suivant la revendication 1, caractérisé par le fait que les tubes de support (7, 8) comportent au moins une subdivision transversale (12), qui divise approximativement par deux la longueur des tubes et au niveau de laquelle les sections séparées (7a, 7b; 8a, 8b) des tubes sont réunies entre elles lors du montage.

3. Dispositif suivant la revendication 2, caractérisé par le fait que les deux tubes de support (7, 8) sont constitués par des sections inférieures respectives (7a, 8a), qui sont reliées à une traverse (9) située au niveau du sol, s'étendent jusqu'à la subdivision transversale (12) et comportent des tubes possédant un profil fermé en coupe traversale, et par des sections supérieurs (7b, 8b), qui se raccordent aux sections inférieures, sont reliées à une traverse (10) située du côté du plafond et comportent des tubes (42) possédant un profil ouvert d'un côté en coupe transversale et délimitant la fente longitudinale (13).

4. Dispositif suivant la revendication 1, caractérisé par le fait que dans la section (7a, 8a) reliée à la traverse (9) située au sol, un tube (8a) est fixé rigidement au pied (9) et l'autre tube (7a) est fixé de manière à être réglable en hauteur.

5. Dispositif suivant la revendication 1, caractérisé par le fait que les tubes de la section (7b, 8b) comportant des fentes longitudinales (13) contiennent chacun une barre de guidage (22, 23), qui est disposée de préférence en position centrée, peut être reliée aux éléments (7a, 8a, 10) situés du côté du sol et du côté du plafond et sur laquelle glissent des manchons de guidage (24, 25) reliés au support (2), et que les barres de guidage (22, 23) sont entourées par un tube enveloppe (42) qui comporte la fente longitudinale (13) et dont le diamètre extérieur correspond à celui des éléments formant cadre (7a, 8a; 10), qui s'y raccordent.

6. Dispositif suivant la revendication 1, caractérisé par le fait que les sections (7b, 8b) des tubes, qui comportent des fentes longitudinales (13, 64), sont formées par un tube enveloppe (44, 61) agencé de manière à former un conduit de guidage, dans lequel peut glisser un élément de guidage (45, 62), sur lequel le support (2) est fixé.

7. Dispositif suivant la revendication 6, caractérisé par le fait que le tube enveloppe (44) est constitué par une tôle enroulée.

8. Dispositif suivant la revendication 5 ou 6, caractérisé par le fait que les éléments de guidage (23, 24, 45, 61) servant à guider le support (2) sont reliés rigidement au support (2), d'un côté de ce dernier, et avec un certain jeu, de l'autre côté.

9. Dispositif suivant la revendication 1, caractérisé par le fait que le support (2) comporte un dispositif (35 à 38) de sécurité contre une chute, qui est constitué par un corps de serrage (36), qui peut être bloqué entre un tube de guidage (22) et une surface oblique (35) reliée au chariot coulissant (2), et est relié à une extrémité (26b) du câble de traction (26) et, sous l'action du ressort de traction ou de compression (26), se dégage de la position de blocage par serrage, dans le cas de la présence du câble de traction, et vient dans la position de blocage par serrage, dans le cas de l'absence du câble de traction.

10. Dispositif suivant la revendication 1, caractérisé par le fait qu'il est prévu, comme élément d'application d'une force, un ressort de traction (27), qui est disposé dans le support (2) et tend un câble de traction (26) circulant sur des galets de renvoi (29, 30), avec une force de traction correspondant à la compensation de poids, et que l'un des galets de renvoi (29, 30) est accouplé à un dispositif extérieur d'entraînement (50, 54, 55, 66).

11. Dispositif suivant la revendication 10, caractérisé par le fait que le galet de renvoi (30) est accouplé à un dispositif de freinage (50 à 53).

12. Dispositif suivant la revendication 11, caractérisé par le fait que l'entraînement et l'action de freinage sont exécutés par l'intermédiaire d'un tambour commun (5) relié au galet de renvoi (30).

13. Dispositif suivant la revendication 12, caractérisé par le fait que sur le galet de renvoi (30) est fixé un tambour (50), sur le bord extérieur duquel s'applique un galet de friction (55) entraîné par un moteur d'entraînement (54), et que dans la zone frontale du tambour (50) se trouvent fixés, sur un support (52) qui ne tourne pas par rapport à ce tambour, deux électroaimants (53), qui coopèrent avec des parties correspondantes du tambour (50), réalisées en un matériau magnétique doux.

14. Dispositif suivant la revendication 1, caractérisé par le fait qu'il est prévu, comme élément d'application d'une force, un moto-réducteur (66) entraînant une poulie à câble (65).

15. Dispositif suivant la revendication 1, caractérisé par le fait qu'il est prévu un autre élément de transmission d'une force, se présentant sous la forme d'un ressort pneumatique (60), dont une extrémité est accrochée au pied (7) et dont l'autre extrémité est accrochée au chariot (2).

16. Dispositif suivant la revendication 4, caractérisé par le fait que le ressort pneumatique (60) est disposé dans un tube enveloppe (61) d'une section (7b) du tube et prend appui, par l'une de ses extrémités, sur un manchon (62) guidé de manière à glisser dans le tube enveloppe (61) et relié au chariot (2).

FIG 1

FIG 2

FIG 3

FIG 5

FIG 8

FIG 6

FIG 7

FIG 4

FIG 9

FIG 10

FIG 11